## Europäisches Patentamt
## European Patent Office
## Office européen des brevets

(19)

(11) Veröffentlichungsnummer: **0 545 307 B1**

(12)

# EUROPÄISCHE PATENTSCHRIFT

(45) Veröffentlichungstag der Patentschrift: **15.02.95**

(51) Int. Cl.⁶: **C07C 15/24**, C07C 2/66, C07C 2/86

(21) Anmeldenummer: **92120298.2**

(22) Anmeldetag: **27.11.92**

(54) **Verfahren zur Herstellung von Monoisopropylnaphthalin.**

(30) Priorität: **30.11.91 DE 4139548**

(43) Veröffentlichungstag der Anmeldung:
**09.06.93 Patentblatt 93/23**

(45) Bekanntmachung des Hinweises auf die
Patenterteilung:
**15.02.95 Patentblatt 95/07**

(84) Benannte Vertragsstaaten:
**DE FR IT**

(56) Entgegenhaltungen:
**WO-A-92/07810**
**DE-A- 1 468 982**
**FR-A- 2 267 999**
**US-A- 3 251 897**
**US-A- 3 631 120**

(73) Patentinhaber: **HOECHST AKTIENGESELL-
SCHAFT**

**D-65926 Frankfurt (DE)**

(72) Erfinder: **Neuber, Marita, Dr.
Schwanheimer Strasse 104
W-6000 Frankfurt am Main (DE)**
Erfinder: **Dettmeier, Udo, Dr.
Behringstrasse 17
W-6233 Kelkheim/Ts (DE)**
Erfinder: **Leupold, Ernst Ingo, Dr.
Auf der Erlenwiese 61
W-6392 Neu-Anspach (DE)**

**Beschreibung**

Die vorliegende Erfindung betrifft ein Verfahren zur Herstellung von Monoisopropylnaphthalin (MIPN) durch Alkylierung von Naphthalin mit Zeolithen des Faujasit-Typs, dessen Kationen durch Erdalkalimetallionen ausgetauscht sind, als Katalysatoren.

2-Monoisopropylnaphthalin (2-MIPN) ist ein wertvolles Zwischenprodukt zur Herstellung von 2-Naphthol nach dem Hock-Verfahren. Bei dem herkömmlichen Verfahren zur Herstellung von 2-Naphthol wird in der Regel das Na-Salz der 2-Naphthalinsulfonsäure in einer Backschmelze zum 2-Naphtholat umgesetzt, aus dem durch anschließendes Ansäuern 2-Naphthol freigesetzt wird. Bei diesem Prozeß fällt eine große Menge an Salzen an. Mit dem Hock-Verfahren wird dieser Nachteil weitgehend vermieden.

Bei der Alkylierung von Naphthalin entstehen neben den einfach alkylierten Produkten auch zwei- und dreifach und bei höheren Umsätzen noch höher alkylierte Naphthaline. In der Monoisopropylnaphthalinfraktion sind 1- und 2-MIPN vorhanden.

In der Diisopropylnaphthalinfraktion (DIPN) ist auch das 2,6-Diisopropylnaphthalin (2,6-DIPN) ein wertvolles Zwischenprodukt für viele hochveredelte Produkte. So dient es unter anderem als Ausgangsstoff zur Herstellung von 2,6-Naphthalindicarbonsäure, 2,6-Dihydroxynaphthalin und 6-Hydroxy-2-naphthalincarbonsäure. Diese Verbindungen werden als Monomere für Hochleistungspolymere eingesetzt.

In der Praxis ist der Bedarf an 2-MIPN wegen der größeren Produktionsmenge an 2-Naphthol wesentlich größer als die benötigte Menge an 2,6-DIPN für die Herstellung von Monomeren. Daher ist es wünschenswert, die Alkylierung so zu lenken, daß eine hohe Selektivität zu MIPN und gleichzeitig ein hoher Anteil an 2-MIPN in der MIPN-Fraktion erreicht wird.

Es ist bekannt, daß für die Alkylierung von Naphthalin zu MIPN und auch DIPN die verschiedensten sauren Katalysatoren eingesetzt werden können. Bei der Alkylierung mit Friedel-Crafts-Katalysatoren, wie $AlCl_3$ oder $BF_3$, entstehen bei der Aufarbeitung Salze und der Katalysator wird zerstört. Außerdem bilden sich im Laufe der Reaktion harzartige Verbindungen, die bei der Aufarbeitung stören. Die Abtrennung der Produkte vom Katalysator ist meist aufwendig. Die Katalysatoren sind sehr korrosiv und kontinuierliche Verfahren sind nur schwer zu realisieren. Daher wurde seit einiger Zeit versucht, diese Art von Katalysatoren durch feste Säuren zu ersetzen.

In der DE-PS 2 644 624 wird ein Verfahren zur Herstellung von 2-MIPN unter Einsatz von $H_3PO_4/SiO_2$ beschrieben. Dabei muß das Naphthalin in großem Überschuß eingesetzt werden, um die Bildung von mehrfach alkylierten Produkten zu vermeiden. Der Naphthalin-Umsatz ist dabei auf höchstens 60 % begrenzt. Das nicht umgesetzte Naphthalin wird abgetrennt und in die Alkylierung zurückgeführt. Eine anschließende Isomerisierung des Reaktionsgemischs ist notwendig, um einen ausreichend hohen Anteil an 2-MIPN zu erhalten. Auch bei der Verwendung von säureaktiviertem Montmorillonit (DE-OS 2 208 363) oder von perfluorierten Sulfonsäureharzen (US-PS 4 288 646) muß Naphthalin im Überschuß eingesetzt werden, um eine Bildung großer Mengen an mehrfach alkylierten Produkten zu vermeiden. Der geringe Umsetzungsgrad des Naphthalins bei all diesen Verfahren stellt einen erheblichen Nachteil dar.

In anderen Arbeiten wird die Möglichkeit der formselektiven Katalyse genutzt, um die 2-Alkyl- und 2,6-Dialkylnaphthaline herzustellen. Formselektive Katalyse bedeutet, daß die Abmessungen der an der Reaktion beteiligten Moleküle oder Übergangszustände in derselben Größenordnung liegen wie die Katalysatorporen. Durch sterische Zwänge kann der Reaktionsverlauf beeinflußt werden. So kann man z.B. bei der Alkylierung von Naphthalin mit Methanol mit Zeolithen des ZSM-5-Typs mit hoher Selektivität die schlanken $\beta$-Isomeren (2-Methyl- und 2,6-Dimethylnaphthalin) erhalten (z.B. D. Fraenkel et al., J. Catal. 110 (1987) 123-132 und EP-OS 280 055). Aber auch bei diesen Verfahren ist der Naphthalin-Umsatz begrenzt, weil die Diffusion der sperrigen Moleküle durch die Zeolithporen stark behindert wird.

Bei dem Verfahren der EP-OS 317 907 wurde ein dealumierter Mordenit-Zeolith für die Alkylierung von Naphthalin mit Propen eingesetzt. Es wurde ein Naphthalin-Umsatz von 97,3 % erzielt. Die Ausbeute an DIPN betrug 68 %, wovon 50 % aus 2,6-DIPN bestanden. Über die Ausbeute an MIPN und die Zusammensetzung dieser Fraktion wurden keine Aussagen getroffen. Auch in dem Verfahren nach der WO 90/03961 wird ein dealumierter Mordenit-Zeolith zur selektiven Herstellung von 2,6-DIPN verwendet. Es wird ausgeführt, daß durch diesen Zeolithtyp der Anteil von 2,6-DIPN an der DIPN-Fraktion größer als der Anteil im thermodynamischen Gleichgewicht und das Verhältnis von 2,6-/2,7-DIPN größer als 1,2 werden. So soll beispielsweise bei 27 %igem Umsatz an Naphthalin der Anteil von 2,6-DIPN 70 % und das Verhältnis von 2,6-/2,7-DIPN 3,0 und das Verhältnis von MIPN/(DIPN + TIPN) 5,1 betragen. Steigt der Umsatz auf 78%, nimmt der Anteil von 2,6-DIPN auf 62 % und das Verhältnis von 2,6-/2,7-DIPN auf 2,6 und das Verhältnis von MIPN/(DIPN + TIPN) auf 1,1 ab. Über die Zusammensetzung der MIPN-Fraktion werden keine Angaben gemacht.

Diese Beispiele zeigen, daß Mordenit-Katalysatoren bei steigendem Umsatz an Naphthalin die Bildung von MIPN gegenüber DIPN stark zurückdrängen.

Auch Faujasite wurden schon als Katalysatoren für die Alkylierung von Naphthalin eingesetzt. In der US-PS 3 251 897 und der DE-PS 1 468 982 wird der Einsatz von Y-Zeolithen, die mit Seltenen Erdmetallionen und/oder Protonen ausgetauscht sind, für die Alkylierung von Naphthalin beschrieben. Der Naphthalinumsatz war jedoch gering und die Katalysatoren wiesen nur kurze Standzeiten auf. In der DE-PS 1 468 982 wird festgestellt, daß $Ca^{2+}$- und $Mg^{2+}$-ausgetauschte X- und Y-Zeolithe die Alkylierung von Aromaten, wie die Ethylierung von Benzol nicht katalysieren, während die mit Ionen der Seltenen Erdmetalle ausgetauschten X- und Y-Zeolithe sehr aktiv für diese Reaktion sind.

In der EP-A 338 292 und der EP-A 432 132 wird die Umsetzung von Naphthalin mit Propen an dealuminierten Y-Zeolithen ($SiO_2/Al_2O_3$ = 10 bis 350) in der Protonenform in Gegenwart von Dekalin und mit Wasserstoff als Trägergas beschrieben. Bei 220 °C und einem Naphthalin-Umsatz von ca. 50 % beträgt dabei die Selektivität zu MIPN zwischen 68 und 75 %, zu DIPN zwischen 24 und 30 % und zu Triisopropylnaphthalinen (TIPN) zwischen 1,5 und 3 %. Der Anteil von 2-MIPN an den MIPN liegt zwischen 91,5 und 93,5 %. Über die Zusammensetzung der DIPN werden keine Aussagen gemacht. Ebenfalls beschrieben ist die Alkylierung von MIPN mit einem Anteil von 93 % 2-MIPN zu DIPN. Der Anteil von 2,6-DIPN an der DIPN-Fraktion beträgt dabei ca. 40 %. Nachteilig bei diesem Verfahren ist, daß bei der Aufarbeitung große Mengen des hochsiedenden inerten Verdünnungsmittels von dem Produktgemisch abgetrennt werden müssen.

Aufgabe der vorliegenden Erfindung war es, ein Verfahren zur Herstellung von MIPN durch Alkylierung von Naphthalin zur Verfügung zu stellen, das die vorstehend beschriebenen Nachteile nicht besitzt und das insbesondere bei hohem Umsatz an Naphthalin eine möglichst hohe Selektivität zu MIPN bei gleichzeitig hohem Anteil an 2-MIPN an der MIPN-Fraktion liefert.

Dies wird gemäß der Erfindung durch den Einsatz von Zeolithen des Faujasit-Typs, dessen Kationen durch Erdalkalimetallionen ausgetauscht sind, als Katalysatoren erreicht. Dabei werden mit hoher Selektivität einfach alkylierte Produkte mit einem hohen Anteil an 2-MIPN gebildet.

Gegenstand der Erfindung ist nun ein Verfahren zur Herstellung von Monoisopropylnaphthalin durch Alkylierung von Naphthalin mit Hilfe von Zeolithen des Faujasit-Typs als Katalysatoren, das dadurch gekennzeichnet ist, daß die Kationen der Zeolithe mit Ionen der Erdalkalimetalle ausgetauscht sind.

Zeolithe sind kristalline Alumosilikate. Si- und Al-Atome sind tetraedrisch von O-Atomen umgeben. Die Tetraeder sind über gemeinsame O-Atome verknüpft und bilden eine Kristallstruktur, die von definierten Poren und Hohlräumen durchzogen ist (vgl. D.W. Breck, Zoelite Molecular Sieves, John Wiley & Sons, (1974), S. 29-185).

Für das erfindungsgemäße Verfahren eignen sich Zeolithe des Faujasit-Typs, dessen Kationen durch Erdalkalimetallionen ausgetauscht sind. Die Struktur dieser Zeolithe zeichnet sich durch Cuboctaeder (Sodalitheinheiten) aus, die durch Doppelsechsringe miteinander verknüpft sind. Es bildet sich ein kubisches Gitter, in dem große Hohlräume (Superkäfige) durch Poren dreidimensional verbunden sind. Die Poren werden durch 12 Si- bzw. Al-Atome begrenzt und haben einen Durchmesser von 0,74 nm. Die Superkäfige haben einen Durchmesser von 1,3 nm. Von allen Zeolithen weisen die Faujasite mit die weitesten Poren und Hohlräume auf (D.W. Breck, a.a.O.). Sie üben daher in ihrem Inneren nur auf sehr große Moleküle und Übergangszustände sterische Zwänge aus.

Es ist überraschend, daß gerade mit diesem Zeolithtyp die erwünschte hohe Selektivität zu den MIPN erzielt werden kann. Der in der EP-A 317 907 beschriebene Mordenit-Katalysator, der in vielen Arbeiten als formselektiver Katalysator für die Umsetzung von einkernigen Aromaten, wie 1-Methyl-2-ethylbenzol (z.B. S.M. Csicsery, in Zeolite Chemistry and Catalysis, J.A. Rabo, Hrsg., ACS Monograph 171 (1976), S. 680 - 713) beschrieben wird, und Porenweiten von 0,65 x 0,70 nm aufweist (Atlas of Zeolite Structure Types, D.H. Olsen und W.M. Meier, Hrsg., Butterworths (1990)), führt zu einer weit stärkeren Bildung von DIPN. Dies gilt auch für andere Zeolithe, deren Poren enger als die von Faujasiten sind, wie Beta- oder EU-1-Zeolithe. Zeolithe vom Typ Beta verfügen über ein dreidimensionales Porensystem. Die Porenradien betragen 0,57 x 0,75 nm und 0,56 x 0,65 nm (J.B. Higgins et al., Zeolites 8 (1988), S. 446 - 452). Die Poren von EU-1-Zeolithen sind nur durch 10 Si- bzw. Al-Atome begrenzt und haben einen Durchmesser von 0,41 x 0,57 nm. Die Poren haben allerdings große seitliche Ausbuchtungen (Atlas of Zeolite Structure Types, D.H. Olsen und W.M. Meier, Hrsg., Butterworths (1990)). In den Beispielen sind einige Ergebnisse der Alkylierung an den verschiedenen Zeolithen gegenübergestellt. Vergleichsversuche mit Lanthan-ausgetauschten Y-Zeolithen ergaben, daß diese hinsichtlich der Alkylierungsreaktion aktiv sind. Allerdings wurden in großem Maße Nebenprodukte (insbesondere Tetralin und Isopropyltetraline sowie hochsiedende Verbindungen) im Alkylat nachgewiesen.

Die erfindungsgemäß verwendeten Zeolithe des Faujasit-Typs sind im Handel erhältlich und können nach bekannten Verfahren mit $SiO_2/Al_2O_3$-Verhältnissen zwischen 2 und etwa 7 hergestellt werden. Die aluminiumreicheren Faujasite werden als X-Zeolithe ($SiO_2/Al_2O_3$ = 2 bis 3) und die aluminiumärmeren als Y-Zeolithe ($SiO_2/Al_2O_3$ = 3 bis 7) bezeichnet. Durch nachträgliche Dealuminierung können allerdings auch höhere $SiO_2/Al_2O_3$-Verhältnisse erreicht werden. Der Aluminiumgehalt kann dabei auf verschiedene Weise verringert werden. Einige Methoden sind z.B. in J. Scherzer, Catalytia Materials; Relationship between Structure and Reactivity, ACS Symp. Ser. 248 (1984), S. 175 - 200, beschrieben. Für das erfindungsgemäße Verfahren sind Faujasite mit $SiO_2/Al_2O_3$-Verhältnissen zwischen 4 und 400, bevorzugt zwischen 5 und 200, besonders geeignet.

Um den Zeolith in eine katalytisch aktive, d.h. saure Form zu überführen, werden die vorhandenen $Na^+$-Ionen durch Ionenaustausch gegen Ionen der Erdalkalimetalle ausgetauscht. Neben den Erdalkalimetallionen können im Zeolith auch geringe Mengen an Seltenen Erdmetallionen und/oder Ammoniumionen oder Protonen vorhanden sein. Dabei ist es zweckmäßig, daß mindestens 50 % der negativen Gitterladungen durch Ionen der Erdalkalimetalle kompensiert werden. Vorzugsweise werden mindestens 95 % der austauschbaren $Na^+$-Ionen durch Ionen der Erdalkalimetalle ersetzt. Als Salze der Erdalkalimetalle, die zum Ionenaustausch verwendet werden, kommen i.a. alle wasserlöslichen Salze in Frage, insbesondere Chloride und Nitrate. Der Zeolith wird dann durch Dehydratisierung (und Deammonisierung bei $NH_4^+$-Formen) bei 200 bis 800°C, bevorzugt bei 250 bis 550°C in die katalytisch aktive Form überführt. Als Katalysatoren sind insbesondere mit $Ca^{2+}$- und $Mg^{2+}$-Ionen ausgetauschte Faujasite (d.h. X- und Y-Zeolithe) geeignet. Sie werden im Folgenden als CaY bzw. CaX oder MgY bzw. MgX bezeichnet.

Die Alkylierungsreaktion kann in der Gasphase, vorzugsweise aber in der Flüssigphase durchgeführt werden. Als Alkylierungsmittel können beispielsweise i-Propanol, Propen, i-Propylchlorid und -bromid eingesetzt werden. Bei der Gasphasenreaktion ist es bevorzugt Naphthalin mit Propen oder i-Propanol umzusetzen. Bei der Alkylierung in der Flüssigphase ist die Verwendung von Propen bevorzugt.

Die Reaktionstemperatur liegt zweckmäßig zwischen etwa 100 und 500°C, bevorzugt zwischen etwa 150 und 300°C. Ein erhöhter Druck ist günstig für den Verlauf der Alkylierung, insbesondere, wenn mit Propen alkyliert wird. Die Reaktion kann bei Unterdruck, Atmosphärendruck oder höherem Druck, z.B. bis etwa 100 bar, bevorzugt zwischen etwa 2 und 20 bar, durchgeführt werden.

Die Alkylierung in der Flüssigphase kann in allen geeigneten Apparaturen durchgeführt werden, am einfachsten diskontinuierlich in einem Rührkessel mit pulverförmigem, in geschmolzenem Naphthalin suspendiertem Katalysator. Das Verfahren kann aber auch kontinuierlich in der Flüssigphase durchgeführt werden. Propen wird dann bei Reaktionstemperatur durch die Suspension geleitet oder bis zum gewünschten Druck aufgepreßt. Zur Erzielung des Reaktionsdruckes können auch inerte Gase wie Stickstoff verwendet werden.

Bezogen auf die Masse an eingesetztem Naphthalin ist es bei diskontinuierlicher Arbeitsweise besonders günstig, zwischen etwa 0,1 und 50 Gew.-% Katalysator, bevorzugt zwischen etwa 1 und 10 Gew.-%, einzusetzen. Die Reaktionsdauer kann je nach Reaktionsbedingungen und gewünschtem Umsatz zwischen etwa 0,5 h und mehreren Tagen, insbesondere zwischen 2 und 10 h, betragen. Nach erfolgter Reaktion kann der Zeolith auf einfache Weise, z.B. durch Filtration, aus dem Reaktionsgemisch abgetrennt werden.

Für die Durchführung der Reaktion in der Gasphase können prinzipiell alle für Gasphasenreaktionen geeigneten Apparaturen verwendet werden. Technisch am einfachsten ist ein Festbett-Strömungsreaktor zu handhaben. Der Katalysator kann dabei in Form von Pellets in den Reaktor eingebaut werden. Für die Herstellung der Pellets kann der Zeolith zusammen mit einem Bindemiffel wie $Al_2O_3$ oder $SiO_2$ oder aber binderfrei verpreßt werden. Als Bindemittel sind vor allem Oxide, Hydroxide oder Hydroxychloride des Aluminiums und die Oxide des Siliziums, Titans und Zirkons sowie Tonmaterialien geeignet.

Das Naphthalin kann in geschmolzenem Zustand oder in einem inerten Lösungsmittel gelöst in den Reaktor eindosiert und vor dem Katalysatorbett verdampft werden oder aber schon vor dem Reaktor in den gasförmigen Zustand überführt und so in den Reaktor geleitet werden. Die Reaktion wird bevorzugt ohne Verwendung von Lösungsmitteln durchgeführt.

I-Propanol kann ebenso wie Naphthalin dosiert werden. Propen wird gasförmig eingeleitet. Die Reaktionsteilnehmer können allein oder im Gemisch mit einem hinsichtlich der Reaktion inerten Gas, wie Wasserstoff oder Stickstoff, eingesetzt werden. Die Durchführung der Reaktion ohne inerte Lösungsmittel oder Gase ist allerdings bevorzugt. Die Produkte werden nach dem Verlassen des Reaktors kondensiert.

Das molare Verhältnis von Naphthalin zum Alkylierungsmittel liegt bei kontinuierlicher Arbeitsweise zweckmäßig im Bereich von etwa 0,1 bis 10, bevorzugt von etwa 0,2 bis 1,2.

Die Verweilzeit der Reaktionsteilnehmer liegt im allgemeinen zwischen etwa 0,05 und 20 s, bevorzugt zwischen 1 und 10 s. Die Raumgeschwindigkeit (LHSV = liquid hourly space velocity = ml Einsatz pro ml Katalysatorvolumen und Stunde) kann vorzugsweise im Bereich von 0,1 und 5 $h^{-1}$ eingestellt werden, wobei

der Bereich zwischen 0,5 und 2 h$^{-1}$ besonders günstig ist.

Der Katalysator ist mehrfach für die Reaktion einsetzbar. Falls er desaktiviert ist, kann er durch Calcinieren in oxidierender Atmosphäre, bevorzugt an Luft, bei etwa 350 bis 800°C, bevorzugt bei etwa 500 bis 600°C, wieder regeneriert werden.

Der Anteil von 2-MIPN im Produktgemisch kann in einem weiteren Verfahrensschritt durch Isomerisierung ebenfalls mit Hilfe von sauren Faujasiten als Katalysatoren noch weiter bis maximal zum Gleichgewichtsanteil erhöht werden (bei 200°C liegt im Gleichgewicht der Anteil von 2-MIPN an der MIPN-Fraktion bei ca. 95 %). Aus diesem Grund ist das Ziel, einen hohen Anteil an 2-MIPN in der MIPN-Fraktion zu erreichen dem Ziel einer hohen Selektivität zu MIPN nachgeordnet. Auch innerhalb der DIPN-Fraktion kann der Anteil an 2,6-DIPN durch nachfolgende Isomerisierung erhöht werden. Im Gleichgewicht bei 200°C beträgt er ca. 42 %. Es ist ein weiterer Vorteil des Verfahrens, daß gleichzeitig zwei wertvolle Produkte (2-MIPN und 2,6-DIPN) hergestellt werden können.

Die bei der Alkylierung gebildeten höheralkylierten Naphthaline können durch Transalkylierung mit Naphthalin wieder zu MIPN umgesetzt werden. Die Transalkylierung ist eine Nebenreaktion der Isomerisierung und kann daher im selben Verfahrensschritt wie die Isomerisierung stattfinden. Erdalkalimetallausgetauschte Zeolithe des Faujasit-Typs sind auch für die Isomerisierung und Transalkylierung von Isopropylnaphthalinen aktiv.

Das Produktgemisch kann zunächst durch Destillation in nicht umgesetztes Naphthalin, MIPN, DIPN und TIPN aufgetrennt werden. Nicht umgesetztes Naphthalin kann entweder wieder für die Alkylierung verwendet oder aber zusammen mit den höher alkylierten Reaktionsprodukten in die Isomerisierungs-/Transalkylierungsstufe eingespeist werden. Aus der MIPN-Fraktion kann 2-MIPN durch Kristallisation, eventuell aus einem Lösungsmittel wie Methanol oder i-Propanol, oder durch Sorption an Molekularsieben abgetrennt werden (siehe z.B. DE-OS 2 517 591). Das an 1-MIPN angereicherte Filtrat kann durch Isomerisierung an verschiedenen Zeolithen wieder in ein an 2-MIPN reiches Gemisch überführt werden (siehe z.B. US-PS 4 026 959). 2,6-DIPN kann ebenfalls durch Kristallisation aus der DIPN-Fraktion abgetrennt werden (siehe z.B. EP-PS 216 009). Eine adsorptive Abtrennung ist z.B. in JP-OS 01 199 921 beschrieben. Der Rest der von 2,6-DIPN weitgehend befreiten DIPN-Fraktion kann zusammen mit Naphthalin durch Transalkylierung wieder zu MIPN umgesetzt werden. Durch nachfolgende übliche Reinigungsschritte können 2-MIPN und 2,6-DIPN bis zum gewünschten Grad weiter gereinigt werden.

Beispiele

Die Na$^+$-Ionen der verwendeten Y-Zeolithe (SiO$_2$/Al$_2$O$_3$ = 5,4) wurden durch Ionenaustausch unter Verwendung von Erdalkalimetallchloriden (bzw. von Seltenen Erdmetallnitraten im Vergleichsbeispiel V4) gegen die entsprechenden Kationen ausgetauscht. Dabei wurde der Zeolith mit der 10fachen Menge 10 %iger wäßriger Lösungen dieser Salze unter Rückfluß 6 bis 7 h behandelt. Dieser Austausch wurde 3 Mal wiederholt. Im Beispiel 1 wurden 85 % der Natriumionen gegen Calciumionen und im Beispiel 2 70 % der Natriumionen gegen Magnesiumionen ausgetauscht.

Aluminiumärmere Y-Zeolithe wurden durch Dealuminierung mit SiCl$_4$ nach der Methode von H. Beyer, beschrieben in H. Beyer und I. Belenykaya, Stud. Surf. Sci. Catal. 5 (1980), S. 203 - 210, hergestellt. Durch wiederholtes Kochen mit der 10fachen Menge 1 n HCl wurden diese aluminiumarmen Zeolithe noch weiter dealuminiert. Nach dieser Behandlung wurden die Zeolithe bei 550°C calciniert.

Die Vergleichszeolithe mit anderen Strukturen wurden durch hydrothermale Synthese nach Vorschriften aus der Literatur hergestellt. Zeolith Beta wurde nach Beispiel 7 aus US-PS 3 308 069 mit einem SiO$_2$/Al$_2$O$_3$-Verhältnis von 23 im Gel synthetisiert. Mordenit kristallisierte bei einer modifizierten Beta-Synthese nach der oben genannten Vorschrift, wobei anstelle von Tetraethylammoniumhydroxid die äquimolaren Mengen Tetraethylammoniumbromid und NaOH eingesetzt wurden. Die Kristallisation von EU-1-Zeolithen ist in G.W. Dodwell et al., Zeolites 5 (1985), S. 153 - 157 beschrieben. Nach der Kristallisation wurden die Zeolithe abfiltriert, getrocknet und an der Luft bei 550°C calciniert. Anschließend wurden sie mit 10 %iger wäßriger NH$_4$NO$_3$-Lösung zwei Mal behandelt und nochmals calciniert. Mordenit wurde zusätzlich, wie die Y-Zeolithe, einmal mit 1 n HCl eine Stunde behandelt und ebenfalls calciniert.

Die Beispiele 1 und 2 und die Vergleichsbeispiele V1 bis V4 wurden in einem Rührkessel durchgeführt. Der Zeolith wurde vor der Reaktion 1 h lang bei 300°C getrocknet und dann pulverförmig in 128 g geschmolzenem Naphthalin suspendiert. Es wurden 5 Gew.-% Zeolith, bezogen auf Naphthalin, eingesetzt. Propen wurde bei Atmosphärendruck mit 6,5 l/h durch die Suspension geleitet. Die Reaktionstemperatur betrug 200°C. Die Versuchsergebnisse sind in der Tabelle zusammengestellt.

Die Ergebnisse der Naphthalinalkylierung mit den erfindungsgemäßen Zeolithen zeigen, daß hohe Selektivitäten zu MIPN $S_{MIPN}$ (über 90 mol-% bei $X_N$ = 20 mol-% Naphthalinumsatz) erzielt werden können.

Die MIPN-Selektivitäten, die mit HMordenit und HBeta (V2 und V3) erreicht werden, sind demgegenüber deutlich vermindert. HEU-1 (V1) liefert zwar ebenfalls eine hohe MIPN-Selektivität, die Anteile an 2-MIPN $A_{2-MIPN}$ an der MIPN-Fraktion sind jedoch deutlich geringer als die in den Beispielen 1 und 2 erzielten Anteile. Mit HMordenit wird, wie in der Literatur beschrieben, ein hoher Anteil an 2,6-DIPN $A_{2,6-DIPN}$ erhalten, allerdings wird neben der geringen Selektivität zu MIPN auch nur ein deutlich geringerer Umsatz nach 6 h Versuchdauer erreicht. Mit Lanthan ausgetauschte Zeolithe (V4) zeigen aufgrund der vielen Nebenprodukte eine geringe Selektivität zu MIPN.

Tabelle

| Bsp. | Zeolith | $SiO_2/Al_2O_3$ | Umsatz an Naphthalin nach 6 h, mol-% | $S_{MIPN}$, mol-% bei $X_N$ mol-% | | | $A_{2\text{-}MIPN}$, % bei $X_N$ mol-% | | | $S_{DIPN}$, mol-% bei $X_N$ mol-% | $A_{2,6\text{-}DIPN}$, % bei $X_N$ mol-% | Ausb. an NP[a] nach 6 h $T^{[b]}$,% | $HS^{[c]}$,% |
|---|---|---|---|---|---|---|---|---|---|---|---|---|---|
| | | | | 20 | 40 | 60 | 20 | 40 | 60 | 40 | 40 | | |
| 1 | CaY | 5,4 | 86 | 92 | 85 | 77 | 83 | 89 | 90 | 14 | 30 | 0,2 | 1,0 |
| 2 | MgY | 5,4 | 68 | 94 | 88 | 82 | 68 | 70 | 63 | 14 | 20 | 0,1 | n.b. |
| V1 | HEU-1 | 49 | 67 | 94 | 85 | 74 | 62 | 60 | 59 | 14 | 18 | 0 | 0,1 |
| V2 | HMordenit | 17 | 38 | 88 | 81 | 66 | 81 | 76 | | 19 | 60 | 0 | <0,1 |
| V3 | HBeta | 12 | 82 | 89 | 78 | 66 | 91 | 89 | 84 | 20 | 41 | 0 | 0,5 |
| V4 | LaY | 5,4 | 86 | 80 | 75 | 67 | 93 | 92 | 91 | 12 | 31 | 1,5 | 9 |

a) NP: Nebenprodukte
b) T: Tetralin und Isopropyltetraline
c) HS: hochsiedende Nebenprodukte
d) n.b.: nicht bestimmt

## Patentansprüche

1. Verfahren zur Herstellung von Monoisopropylnaphthalin durch Alkylierung von Naphthalin mit Hilfe von Zeolithen des Faujasit-Typs als Katalysatoren, dadurch gekennzeichnet, daß die Kationen der Zeolithe mit Ionen der Erdalkalimetalle ausgetauscht sind.

**2.** Verfahren nach Anspruch 1, dadurch gekennzeichnet, daß die Erdalkalimetallionen $Mg^{2+}$- und/oder $Ca^{2+}$-Ionen sind.

**3.** Verfahren nach Anspruch 1 oder 2, dadurch gekennzeichnet, daß der Zeolith ein $SiO_2/Al_2O_3$-Verhältnis von 4 bis 400, bevorzugt von 5 bis 250, aufweist.

**4.** Verfahren nach einem oder mehreren der Ansprüche 1 bis 3, dadurch gekennzeichnet, daß in den Zeolithen des Faujasit-Typs mindestens 50 % der negativen Gitterladungen durch Erdalkalimetallionen kompensiert werden.

**5.** Verfahren nach einem oder mehreren der Ansprüche 1 bis 3, dadurch gekennzeichnet, daß mindestens 95 % der austauschbaren $Na^+$-Ionen durch Ionen der Erdalkalimetalle ausgetauscht sind.

**6.** Verfahren nach einem oder mehreren der Ansprüche 1 bis 5, dadurch gekennzeichnet, daß die Reaktion in der flüssigen Phase durchgeführt wird.

**7.** Verfahren nach einem oder mehreren der Ansprüche 1 bis 6, dadurch gekennzeichnet, daß die Reaktion bei Temperaturen von 100 bis 500 °C, bevorzugt von 150 bis 300 °C, durchgeführt wird.

**8.** Verfahren nach einem oder mehreren der Ansprüche 1 bis 7, dadurch gekennzeichnet, daß die Reaktion bei einem Druck bis 100 bar, bevorzugt 2 bis 20 bar, durchgeführt wird.

**9.** Verfahren nach einem oder mehreren der Ansprüche 1 bis 8, dadurch gekennzeichnet, daß als Alylierungsmittel i-Propylbromid, i-Propylchlorid, Propen oder i-Propanol eingesetzt werden.

**10.** Verfahren nach einem oder mehreren der Ansprüche 1 bis 9, dadurch gekennzeichnet, daß das Naphthalin bei kontinuierlicher Arbeitsweise im molaren Verhältnis von 0,1 bis 10, bevorzugt von 0,2 bis 1,2, bezogen auf das Alkylierungsmittel, eingesetzt wird.

**11.** Verfahren nach einem oder mehreren der Ansprüche 1 bis 10, dadurch gekennzeichnet, daß bei diskontinuierlicher Arbeitsweise, bezogen auf die Masse an eingesetztem Naphthalin, 0,1 bis 50 Gew.-%, bevorzugt 1 bis 10 Gew.-%, Katalysator eingesetzt werden.

**Claims**

**1.** A process for the preparation of monoisopropylnaphthalene by alkylation of naphthalene with the aid of zeolites of the faujasite type as catalysts, wherein the cations of the zeolites have been replaced by alkaline earth metal ions.

**2.** The process as claimed in claim 1, wherein the alkaline earth metal ions are $Mg^{2+}$ and/or $Ca^{2+}$ ions.

**3.** The process as claimed in claim 1 or 2, wherein the zeolite has a $SiO_2/Al_2O_3$ ratio of from 4 to 400, preferably from 5 to 250.

**4.** The process as claimed in one or more of claims 1 to 3, wherein at least 50% of the negative lattice charges in the zeolites of the faujasite type are compensated by alkaline earth metal ions.

**5.** The process as claimed in one or more of claims 1 to 3, wherein at least 95% of the exchangeable $Na^+$ ions have been replaced by alkaline earth metal ions.

**6.** The process as claimed in one or more of claims 1 to 5, wherein the reaction is carried out in the liquid phase.

**7.** The process as claimed in one or more of claims 1 to 6, wherein the reaction is carried out at temperatures of from 100 to 500 °C, preferably of from 150 to 300 °C.

**8.** The process as claimed in one or more of claims 1 to 7, wherein the reaction is carried out under a pressure of up to 100 bar, preferably from 2 to 20 bar.

9. The process as claimed in one or more of claims 1 to 8, wherein the alkylating agent used is i-propyl bromide, i-propyl chloride, propene or i-propanol.

10. The process as claimed in one or more of claims 1 to 9, wherein, in the case of a continuous procedure, naphthalene is used in a molar ratio of 0.1 to 10, preferably of 0.2 to 1.2, with respect to the alkylating agent.

11. The process as claimed in one or more of claims 1 to 10, wherein, in the case of a discontinuous procedure, from 0.1 to 50% by weight, preferably from 1 to 10% by weight, of catalyst are used with respect to the mass of naphthalene employed.

**Revendications**

1. Procédé pour la préparation de monoisopropylnaphtalène par alkylation du naphtalène au moyen de zéolithes du type faujasite comme catalyseurs, caractérisé en ce que les cations des zéolithes sont échangés avec des ions des métaux alcalino-terreux.

2. Procédé selon la revendication 1, caractérisé en ce que les ions de métaux alcalino-terreux sont $Mg^{2+}$ et/ou $Ca^{2+}$.

3. Procédé selon la revendication 1 ou 2, caractérisé en ce que la zéolithe présente un rapport $SiO_2/Al_2O_3$ de 4 à 400, de préférence de 5 à 250.

4. Procédé selon une ou plusieurs des revendications 1 à 3, caractérisé en ce que, dans les zéolithes du type faujasite, au moins 50 % des charges négatives du réseau sont compensées par des ions alcalino-terreux.

5. Procédé selon une ou plusieurs des revendications 1 à 3, caractérisé en ce que au moins 95 % des ions $Na^+$ échangés sont échangeables avec des ions des métaux alcalino-terreux.

6. Procédé selon une ou plusieurs des revendications 1 à 5, caractérisé en ce que la réaction est effectuée en phase liquide.

7. Procédé selon une ou plusieurs des revendications 1 à 6, caractérisé en ce que la réaction est effectuée à des températures de 100 à 500 °C, de préférence de 150 à 300 °C.

8. Procédé selon une ou plusieurs des revendications 1 à 7, caractérisé en ce que la réaction est effectuée à une pression allant jusqu'à 100 bars, de préférence de 2 à 20 bars.

9. Procédé selon une ou plusieurs des revendications 1 à 8, caractérisé en ce que, comme agent d'alkylation, on utilise le bromure d'i-propyle, le chlorure d'i-propyle, le propène ou l'i-propanol.

10. Procédé selon une ou plusieurs des revendications 1 à 9, caractérisé en ce que le naphtalène est utilisé dans un mode opératoire continu dans des rapports molaires de 0,1 à 10, de préférence de 0,2 à 1,2, calculé sur l'agent d'alkylation.

11. Procédé selon une ou plusieurs des revendications 1 à 10, caractérisé en ce que, pour un mode opératoire discontinu, on utilise de 0,1 à 50 % en poids, de préférence de 1 à 10 % en poids, de catalyseur, calculé sur la masse du naphtalène utilisé.